# EUROPÄISCHE PATENTANMELDUNG

(11) **EP 1 410 763 A1**
(43) Veröffentlichungstag der Anmeldung: **21.04.2004**
(21) Anmeldenummer: 02022882.1
(22) Anmeldetag: 14.10.2002
(51) Int. Cl.: A61B 17/16

(54) **Chirurgisches Bearbeitungsinstrument**

(71) Anmelder: Waldemar Link (GmbH & Co.), 22339 Hamburg (DE)
(72) Erfinder: Keller, Arnold, 23863 Kayhude (DE)
(74) Vertreter: Glawe, Delfs, Moll & Partner

(57) **Zusammenfassung**

Chirurgisches Bearbeitungsinstrument, insbesondere Fräser oder Bohrer, der in einem abgewinkelten Schaft (2) eine winkeltolerante Welle (4) führt. Der die Abwinkelung (3) bildende Abschnitt des Schafts (2) wird von einer längs geteilten und leicht zerlegbaren Hülse (22) gebildet. Zweckmäßigerweise steht eine Mehrzahl solcher Hülsen (22) mit unterschiedlicher Abwinkelung zur Verfügung. Die Hülsen werden durch Ringteile (24, 25) zusammengehalten.

## Beschreibung

Es sind chirurgische Fräser bekannt (US-A-6364910), die am Ende eines langgestreckten, abgewinkelten Schafts gelagert sind, um auch in engen Operationsöffnungen beispielsweise zum Ausfräsen einer Hüftgelenkpfanne eingesetzt werden zu können. Der Schaft wird von einem Rohr gebildet, in welchem eine flexible Welle gelagert ist. Zum Zwecke der Reinigung und Sterilisierung muß das Instrument zerlegbar sein. Die Zerlegbarkeit bekannter Instrumente läßt zu wünschen übrig.

Der Erfindung liegt daher die Aufgabe zu Grunde, ein Instrument der oben genannten Art zu schaffen, dessen Schaft leicht zerlegbar ist und dennoch zur Übertragung der mit dem Fräsvorgang verbundenen Kräfte in der Lage ist.

Die erfindungsgemäße Lösung besteht in den Merkmalen des Anspruchs 1. Demnach ist vorgesehen, daß der Schaft oder mindestens der darin die Abwinklung bildende Abschnitt von einer längs geteilten und leicht zerlegbaren Hülse gebildet ist. Zum Zusammenhalten der Hülsenteile ist zweckmäßigerweise ein Ringteil vorgesehen, der in Längsrichtung auf die Hülsenteile aufzuschieben ist. Ein solcher Ringteil kann von dem Ende eines der Hülse benachbarten Teils des Schafts und/oder des Instrumentenkopfs gebildet sein. Dadurch wird nicht nur die Zusammenhaltung der Hülsenteile sondern auch die sichere und richtungsgleiche Kraftübertragung von dem Schaft auf die Hülse und von der Hülse auf den Instrumentenkopf bzw. das Werkzeug gewährleistet. Zum Zerlegen genügt es, die Ringe von der Hülse abzuziehen und die längs geteilten Hülsenteile seitlich abzunehmen. Dann liegen die Welle und die zu ihrer Lagerung gegebenenfalls in der Hülse vorgesehene Lager frei.

Der winkeltolerante Teil der Welle wird zweckmäßigerweise von einem Kardangelenk gebildet, das den Vorteil hat, daß es gewünschtenfalls zu Übertragung der Längskräfte herangezogen werden kann, sofern seine Teile mindestens radial gelagert sind. Jedoch soll auch die Verwendung einer biegsamen Welle nicht ausgeschlossen werden. Die Übertragung der Längskräfte obliegt dann dem Schaft.

Nach einem besonderen Merkmal der Erfindung, das Schutz gegebenenfalls unabhängig von den Merkmalen der Ansprüche 1 bis 5 verdient, steht eine Mehrzahl von Schäften bzw. Hülsen mit unterschiedlicher Abwinklung zur Verfügung. Dies erlaubt es, das Instrument den jeweiligen Zugangsverhältnissen oder den Wünschen des Operateurs anzupassen.

Zweckmäßigerweise ist der Winkel der Abwinklung nicht größer als 30° und vorzugsweise in der Größenordnung von 20°, um dem Operateur die Übertragung aller erforderlichen Kräfte allein aufgrund einer am freien Ende des Schafts angeordneten Handhabe, die beispielsweise von einem Antriebsmotor gebildet ist, zu erlauben. Statt dessen oder zusätzlich kann nach der Erfindung an dem Schaft ein Griffteil quer zu Schaftrichtung angeordnet sein, der zweckmäßigerweise leicht lösbar und verstellbar ist, damit der Operateur die Kräfte entweder auch allein über diesen Griffteil ausüben oder durch Benutzung der am freien Ende des Schafts angeordneten Handhabe und dieses Griffteils sicherer steuern kann. Zweckmäßigerweise ist die Anordnung so getroffen, daß der Griffteil in Richtung der Bearbeitungsachse eingestellt werden kann, weil in dieser Richtung die Vorschubkräfte hauptsächlich aufgebracht werden.

Die Erfindung wird im folgenden näher unter Bezugnahme auf die Zeichnung erläutert, deren vorteilhaftes Ausführungsbeispiel veranschaulicht:
- Figur 1: Eine Gesamtansicht des Instruments, teilweise geschnitten,
- Figur 2: den Instrumentenkopf mit Kardangelenk ohne Hülse,
- Figur 3: einen Schnitt durch die Hülse und die darin gelagerten Teile.

Das Instrument besteht aus einem Fräswerkzeug 1, einem Schaft 2 mit Abwinklung 3, der eine Antriebswelle 4 enthält, und einem Antriebsmotor 5, der mit dem freien Ende 6 der Antriebswelle 4 verbunden ist. Ferner kann der Schaft 2 mit einem zusätzlichen Griffteil 7 verbunden werden.

Das Fräswerkzeug 1, das beispielsweise zum Räumen der Hüftpfanne vor dem Einsetzen eines Pfannenimplantats vorgesehen ist und insoweit bekannten Werkzeugen gleicht, kann mittels einer geeigneten Kupplung 12 auf die Werkzeugwelle 13 lösbar aufgesetzt werden. Die Werkzeugwelle 13 ist mit einem Kardanabtriebsteil 14 fest verbunden, der über ein Kardanzwischenstück 15 mit Gelenkstiften 16 und 17 mit dem Kardanantriebsteil 18 gelenkig verbunden ist. Die Funktion eines Kardangelenks bedarf keiner Erläuterung, da sie bekannt ist. Es genügt festzustellen, daß von dem Kardanantriebsteil 18, der um die Achse 19 drehend angeordnet ist, der Kardanabtriebsteil 14 in Drehung um eine andere Achse 10 versetzt werden kann, wobei die Achsen 19 und 10 um einen wählbaren Winkel 21 voneinander abweichen können.

Kardanantriebsteil 18 und Kardabtriebsteil 14 haben zylindrische Außenflächen. Über diese sind sie drehbar gelagert in einer Hülse 22, die dafür geeignete Lagerbohrungen 23, 24 enthält, deren Richtungen sich um den Winkel 21 der gewünschten Abwinklung unterscheiden. Ihre Achsen treffen sich im Kreuzungspunkt der Kardanstifte 16 und 17.

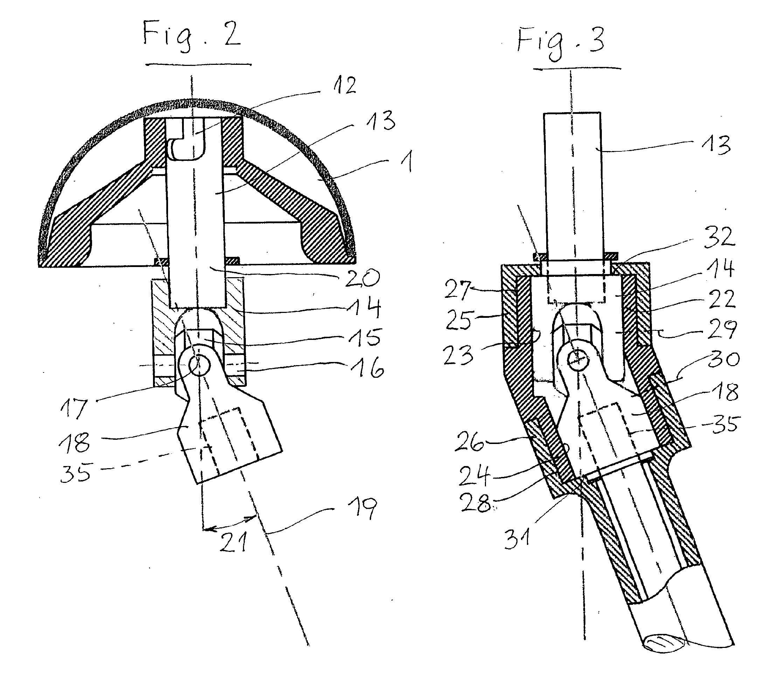

Die Hülse 22 ist in der Zeichenebene geteilt in zwei spiegelbildlich gleiche Hälften. In der in Figur 3 dargestellten Lage werden sie durch zwei Ringe 25, 26 gehalten, deren zylindrische Bohrungen 27, 28 mit den zylindrischen Außenflächen 27, 28 der Hülse 22 zusammenwirken. Die Ringe 25, 26 können in Längsrichtung auf die Hülse 22 aufgeschoben werden und werden in der gewünschten Lage gesichert durch nicht näher dargestellte Schrauben, deren Mittellinien bei 29, 30 angedeutet sind.

Die Ringe 24, 25 bilden innere, stirnseitige Flanschflächen 21, 22, mit denen sie mit den entsprechenden Stirnflächen der ab- und antriebsseitigen Kardanteile 14, 18 zur Bildung von Axiallagern zusammenwirken.

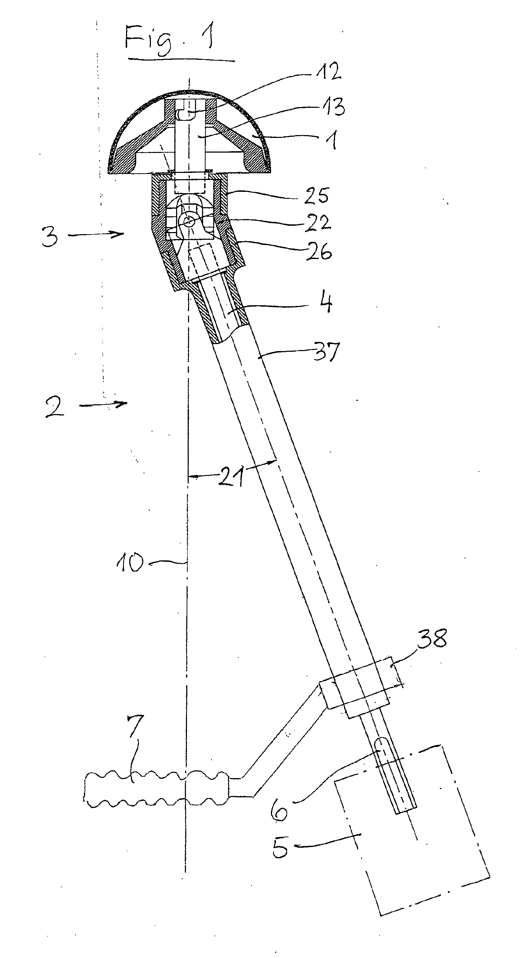

Der antriebsseitige Kardanteil 18 enthält eine Bohrung 35, deren Querschnitt passend mit dem Ende der Welle 4 zusammenwirkt. Der Ring 14 ist einstückig mit dem Schaft 37 verbunden. Auf das andere Ende 6 der Welle 4 kann ein geeigneter Antriebsmotor 5 aufgesetzt werden, der in Figur 1 strichpunktiert angedeutet ist. Er bildet - wie bekannt - eine Handhabe für das Fräsinstrument.

Bei der Montage des Instruments werden zunächst die die Hülse 22 bildenden Halbschalen um das Kardangelenk 14 bis 18 geschlossen und wird der Ring 25 aufgeschoben und mittels der Schraube 29 gesichert. Anschließend wird das Ende der Welle 4 in die Bohrung 25 des Kardanantriebsteils 18 eingeführt. Gleichzeitig oder anschließend kann der Ring 24 über die Hülse 22 geschoben und mittels der Schraube 20 gesichert werden. Wenn die Kupplung 12 mit dem Fräswerkzeug 1 und das Wellenende 6 mit dem Antriebsmotor 5 verbunden ist, ist die Montage beendet. Die Demontage vollzieht sich in umgekehrter Reihenfolge.

Man erkennt, daß der den Motor 5 als Handhabe benutzende Operateur das Instrument leicht ausrichten und die für den Fräsvorgang nötigen Vorschubkräfte auf das Werkzeug 1 ausüben kann. Diese Kräfte werden von dem Motor 5 bzw. der Handhabe auf die Welle 4 und von dieser über das Kardangelenk auf die werkzeugseitige Abtriebswelle 13 übertragen. Dank der Führung der Kardanteile 14 und 18 innerhalb der Hülse 22 können diese ihre vorgeschriebene Aufrichtung unter der Einwirkung dieser Vorschubkräfte nicht verändern.

Wünscht der Operateur für die Abwinklung 3 einen anderen Betrag, so wechselt er die Hülse 22 gegen eine andere aus, die eine entsprechend andere Abwinklung aufweist. Das Kardangelenk 14, 18 kann sich auf diesen anderen Winkel ohne weiteres einstellen.

Reicht dem Operateur die ihm beim Motor 5 zur Verfügung stehende Handhabe nicht aus, so kann er mit dem Schaft 37 einen weiteren Griffteil 7 mittels eines geeigneten Verbindungsstücks 38 lösbar verbinden. Zweckmäßigerweise ist die Anordnung so getroffen, daß er die Lage dieses Griffteils 7 im Verhältnis zum Instrument und der anderen Handhabe 5 unterschiedlich einstellen kann. Zweckmäßig ist es, wenn es eine Einstellungsmöglichkeit gibt, bei der die Werkzeugachse 10 durch den Griffteil 7 oder den Bereich zwischen diesem Griffteil 7 und der weiteren Handhabe 5 hindurchgeht. Das erlaubt dem Operateur auch ohne Sichtkontakt mit dem Fräser 1 die Abschätzung seiner jeweiligen Position. Außerdem kann er die Vorschubkraft genau in Richtung der Fräserachse 10 einsetzen.

## Patentansprüche

1. Chirurgisches Bearbeitungsintrument, insbesondere Fräser oder Bohrer, mit einem abgewinkelten Schaft (2) der eine winkeltolerante Welle führt, **dadurch gekennzeichnet, daß** mindestens der die Abwinkelung bildende Abschnitt (3) des Schafts (2) von einer längs geteilten und leicht zerlegbaren Hülse (22) gebildet ist.

2. Instrument nach Anspruch 1 **dadurch gekennzeichnet, daß** zum Zusammenhalten der Hülsenteile (22) mindestens ein Ringteil (24, 25) vorgesehen ist, der in Längsrichtung auf die Hülsenteile (22) aufzuschieben ist.

3. Instrument nach Anspruch 2 **dadurch gekennzeichnet, daß** der Ringteil (24) von dem Ende eines der Hülse (22) benachbarten Teils (37) des Schafts (2) gebildet ist.

4. Instrument nach einem der Ansprüche 1 bis 3 **dadurch gekennzeichnet, daß** die Hülse (22) und/oder der ihr benachbarte Teil (37) des Schafts (2) Lager (27, 28, 31, 32) für die Welle (4) enthält.

5. Instrument nach einem der Ansprüche 1 bis 4 **dadurch gekennzeichnet, daß** die Welle (4) als winkeltoleranten Teil ein Kardangelenk (14, 15, 18) umfaßt.

6. Instrument, insbesondere nach einem der Ansprüche 1 bis 5, **dadurch gekennzeichnet, daß** eine Mehrzahl von Schäften (2) bzw. Hülsen (22) mit unterschiedlicher Abwinkelung (3) verfügbar ist.

7. Instrument nach einem der Ansprüche 1 bis 6, **dadurch gekennzeichnet, daß** der Winkel (21) der Abwinkelung (3) nicht größer als (30)° ist.

8. Instrument nach einem der Ansprüche 1 bis 7, **dadurch gekennzeichnet, daß** an dem Schaft (2) ein Griffteil (7) quer zur Schaftrichtung angeordnet ist.

9. Instrument nach Anspruch 8, **dadurch gekennzeichnet, daß** der Griffteil (7) leicht lösbar ist.

10. Instrument nach Anspruch 8 oder 9, **dadurch gekennzeichnet, daß** der Griffteil (7) verstellbar ist.

11. Instrument nach einem der Ansprüche 1 bis 10, **dadurch gekennzeichnet, daß** das mit dem freien Ende der Welle (4) verbundene Antriebsorgan (5) eine Handhabe bildet oder aufweist.
